Europäisches Patentamt

European Patent Office

Office européen des brevets

(19)

(11) Veröffentlichungsnummer : **0 413 171 B1**

(12) **EUROPÄISCHE PATENTSCHRIFT**

(45) Veröffentlichungstag der Patentschrift :
26.05.93 Patentblatt 93/21

(51) Int. Cl.$^5$ : **A61K 31/485**, A61K 9/48

(21) Anmeldenummer : **90114108.5**

(22) Anmeldetag : **24.07.90**

(54) **Mittel zur Behandlung von schweren Schmerzzuständen und Verfahren zu ihrer Herstellung.**

(30) Priorität : **17.08.89 DE 3927113**

(43) Veröffentlichungstag der Anmeldung :
**20.02.91 Patentblatt 91/08**

(45) Bekanntmachung des Hinweises auf die
Patenterteilung :
**26.05.93 Patentblatt 93/21**

(84) Benannte Vertragsstaaten :
**AT BE CH DE DK ES FR GB GR IT LI LU NL SE**

(56) Entgegenhaltungen :
**WO-A-85/04589**
**US-A- 4 571 400**

(73) Patentinhaber : **Dolorgiet GmbH & Co. KG**
**Otto-von-Guericke-Str. 1**
**W-5205 St. Augustin 3 (DE)**

(72) Erfinder : **Löhner, Manfred**
**Blücherstrasse 47**
**W-5300 Bonn 1 (DE)**
Erfinder : **Posselt, Klaus, Dr.**
**Rodderbergstrasse 62**
**W-5300 Bonn 2 (DE)**
Erfinder : **Vögtle-Junkert, Ute, Dr. med.**
**In-der-Asbach 10**
**W-5305 Alfter (DE)**
Erfinder : **Wagener, Hans H., Prof. Dr. med.**
**Breslauer Strasse 76**
**W-5309 Meckenheim (DE)**

(74) Vertreter : **Werner, Hans-Karsten, Dr. et al**
**Deichmannhaus am Hauptbahnhof**
**W-5000 Köln 1 (DE)**

EP 0 413 171 B1

## Beschreibung

Gegenstand der vorliegenden Erfindung sind Mittel zur Behandlung von schweren Schmerzzuständen, insbesondere von Tumorschmerzen und postoperativen Schmerzen sowie Verfahren zu ihrer Herstellung.

Aus der EP-A-85 111 013.0 (0 178 436) sind Ibuprofen enthaltene Weichgelatinekapseln und Verfahren zu ihrer Herstellung bekannt. Diese Weichgelatinekapseln haben sich bewährt zur Bekämpfung akuter Schmerzen mit raschem Wirkungseintritt, da sie den Wirkstoff Ibuprofen rasch freisetzen und damit eine gute Bioverfügbarkeit des Wirkstoffes erreichen.

Cooper et al. haben die analgetische Wirksamkeit von Kombinationen von Ibuprofen und Codein untersucht, und dabei festgestellt, daß Codein zwar die analgetische Wirksamkeit von Ibuprofen etwas erhöht, jedoch war diese Erhöhung nur so gering, daß man sie für statistisch nicht ausreichend signifikant angesehen hat, vergl. Pharmacotherapy 1982; 2:162-167.

Abraham Sunshine et al. zitieren Untersuchungen über die Schmerzbekämpfung bei der Extraktion der hinteren Backenzähne (Giles und Pickvance; Frame et al.) und nach Episiotomie (Norman et al.). Auch hier wurde gefunden, daß die gleichzeitige Verabreichung von Ibuprofen und Codein zwar effektiver als die von Ibuprofen allein ist, statistisch signifikante Unterschiede aber waren nicht sichtbar. Nach eigenen Untersuchungen von Abraham Sunshine et al. zeigte nur die hohe Dosis von Ibuprofen und Codein (400 + 60 mg) bei Episiotomie (Einschnitt zur Geburtserleichterung) eine bessere analgetische Wirksamkeit gegenüber Ibuprofen allein. Niedriger dosierte Kombinationen waren nicht besser als Ibuprofen (Clin. Pharm. Ther. 1987; 42: 374-380).

Ibuprofen ist somit ein gut verträgliches und gut wirksames Analgetikum, welches jedoch nur bei leichteren und akuten Schmerzen eingesetzt wird. Zur Behandlung von schweren Schmerzzuständen, insbesondere von Tumorschmerzen und postoperativen Schmerzen, ist es nach wie vor notwendig, Morphium oder Morphin-Derivate zu applizieren. Es ist bekannt, daß Morphium und Morphin-Derivate zu einer raschen Gewöhnung führen und daher nur dann zur Anwendung kommen sollten, wenn alle übrigen Analgetika ihre Wirksamkeit verloren haben.

Die Erfindung hat sich somit die Aufgabe gestellt, ein Mittel zur Behandlung von schweren Schmerzzuständen, insbesondere von Tumorschmerzen und postoperativen Schmerzen zu entwickeln, welches auf Morphium und Morphin-Derivate verzichtet. Überraschenderweise kann diese Aufgabe gelöst werden durch Mittel bestehend aus Weichgelatinekapseln enthaltend 30 bis 50 Gewichtsteile Ibuprofen und 1,5 bis 4 Gewichtsteile Codein und/oder seine physiologisch verträglichen Salze, teilweise gelöst und teilweise suspendiert in 68,5 bis 46 Gewichtsteilen Polyoxyethylen-polyoxypropylen-Diol oder einem Gemisch aus 30 bis 76 Gewichtsteilen Polyoxyethylen-polyoxypropylen-Diol oder Polyethylenglykol oder Polypropylenglykol und 7 bis 40 Gewichtsteilen eines physiologisch verträglichen Tensids.

Ein Vergleich mit Dragees enthaltend die gleiche Menge Ibuprofen und Codein hat die Ergebnisse von Cooper et al. und Sunshine et al. bestätigt und gezeigt, daß eine derartige Kombination ungeeignet ist, schwere Schmerzzustände, insbesondere Tumorschmerzen und postoperative Schmerzen, zu behandeln. Das erfindungsgemäße Mittel zeigt hingegen eine überraschend gute Wirkung bei vielen Patienten mit sehr raschem Wirkungseintritt einerseits und relativ langer Wirkungsdauer andererseits, so daß erst relativ spät nachdosiert werden muß. Es hat sich somit gezeigt, daß in sehr vielen Fällen auf die Applikation von Morphium und Morphin-Derivaten verzichtet werden kann oder aber diese Mittel erst wesentlich später zum Einsatz kommen müssen.

Die erfindungsgemäßen Weichgelatinekapseln enthalten somit neben dem teilweise gelösten und teilweise suspendierten Ibuprofen im allgemeinen die gleichen Lösungsmittel wie die Ibuprofen enthaltenen Weichgelatinekapseln gemäß EP-A-85 111 013.0 (0 178 436). Vorzugsweise enthalten sie somit auch zusätzlich bis zu 3 Gewichtsteilen 1,2-Propylenglykol. Als Tensid enthalten sie vorzugsweise Polyoxyethylen-glycerol-tri-hydroxystearat, Polyoxyethylen-($C_{12-18}$)-fettalkoholether, Polyoxyethylenstearat, Polyoxyethylen-sorbitan-mono($C_{12-18}$)-fettsäureester, Polyoxyethylen-polyoxypropylen-Diol oder Gemische derselben.

Auch die Herstellung der erfindungsgemäßen Mittel erfolgt in ähnlicher Weise wie die Herstellung von Ibuprofen enthaltenden Weichgelatinekapseln, nämlich in der Weise, daß 15 bis 30 Gewichtsteile Ibuprofen bei Temperaturen zwischen 45 und 65°C in 70 bis 85 Gewichtsteilen Polyoxyethylen-Polyoxypropylen-Diol oder in einem Gemisch aus 30 bis 76 Gewichtsteilen Polyoxyethylen-polyoxypropylen-Diol oder Polyethylenglykol oder Polypropylenglykol und 7 bis 40 Gewichtsteilen eines physiologisch verträglichen Tensids gelöst, auf Raumtemperatur abgekühlt, in der Lösung nach dem Abkühlen zusätzlich bis zu 40 Gewichtsteile Ibuprofen und 1,5 bis 4 Gewichtsteile Codein und/oder seine physiologisch verträglichen Salze suspendiert werden und das Gemisch in an sich bekannter Weise in Weichgelatinekapseln eingearbeitet wird.

Besonders bevorzugt sind Mittel, welche 40 bis 45 Gewichtsteile Ibuprofen und 2,5 bis 3 Gewichtsteile Codeinphosphat·0,5 $H_2O$ enthalten, welche teilweise gelöst und teilweise suspendiert sind, in einem Gemisch aus 86 bis 90 Gewichtsteilen Polyoxyethylen-polypropylen-Diol, 8 bis 12 Gewichtsteilen Polyoxyethylen-(40)-glycerol-tri-hydroxystearat und 1,5 bis 2,5 Gewichtsteilen Propylenglykol.

Sie werden hergestellt, indem zunächst 20 bis 25 Gewichtsteile Ibuprofen in einem warmen Gemisch aus 86 bis 90 Gewichtsteilen Polyoxyethylen-polyoxypropylen-Diol, 8 bis 12 Gewichtsteilen Polyoxyethylen-(40)-glycerol-tri-hydroxystearat und 1,5 bis 2,5 Gewichtsteilen 1,2-Propylenglykol gelöst, dann auf Raumtemperatur abgekühlt und in dem Gemisch weitere 20 bis 25 Gewichtsteile Ibuprofen und 2,5 bis 3 Gewichtsteile Codein-phosphat·0,5 $H_2O$ suspendiert werden und in Weichgelatinekapseln abgefüllt werden.

Diese Mittel haben sich deshalb besonders gut bewährt, da sie eine besonders hohe Wirkstoffmenge pro Gewichts- und Volumeneinheit Weichgelatinekapsel enthalten und dennoch die Wirkstoffe rasch und mit hoher Bioverfügbarkeit zur Verfügung stellen. Die Größe der Kapseln bzw. die Anzahl der Kapseln, die einzunehmen sind, liegen somit in einem möglichst niedrigem Bereich.

In den nachfolgenden Beispielen sind die Herstellung einer bevorzugten Rezeptur beschrieben sowie die Vergleichsversuche mit Dragees, welche die gleiche Menge Wirkstoffe enthalten wie die erfindungsgemäßen Weichgelatinekapseln.

## Beispiel 1

Man stellt unter Rühren bei 60 bis 70°C eine Mischung aus 196,65 g Polyoxyethylen-polyoxypropylen-Diol 1900 (Pluronic[R] L 35, Wyandotte Chemicals Corp.), 21,60 g Polyoxyethylen-(40)-glycerol-tri-hydroxystearat (Cremophor[R] RH 40, BASF) und 4,50 g 1,2-Propylenglykol her, kühlt auf 50°C ab und rührt portionsweise 90,00 g Ibuprofen ein. In dieser Lösung werden bei 15 bis 20°C weitere 90,00 g feinverteiltes Ibuprofen und 11,25 g Codeinphosphat·0,5 $H_2O$ suspendiert. Die so erhaltene Suspension wird in Weichgelatinekapseln geeigneter Größe eingearbeitet. 0,920 g Suspension enthalten 400 mg Ibuprofen und 25 mg Codeinphosphat·0,5 $H_2O$.

## Beispiel 2

Die gemäß Beispiel 1 hergestellten Weichgelatinekapseln wurden in einer vergleichenden Studie an insgesamt 20 Patienten bei Schmerzzuständen, wie Tumorschmerzen, getestet, und zwar im Vergleich zu Ibuprofen-Codein-Dragees mit gleichem Gehalt an Wirkstoff.

Die Diagnosen umfaßten Mamma-, Bronchial- und Lungengrund-Karzinom mit Knochenmetastasen, Oesophagus-, Zungengrund-Karzinom mit Lymphmetastasen, Colon-Karzinom mit Lebermetastasen, Plasmacytom mit Kompressionsfraktur, Sigma-Karzinom u.a.

Aus den Kalenderdaten am 1., 2. und 3. Behandlungstag wurde mit einem Programm die Behandlungsdauer in Tagen nach dem 2. und 3. Untersuchungstag und mit einem weiteren Programm aus den Einnahme-Uhrzeiten die Zeit der Nach-Dosierung in Stunden bezogen auf die Erst-Dosierung am gleichen Untersuchungstag berechnet.

Für die metrischen patienten- und versuchsbeschreibenden Parameter sowie die Mindestwirkzeit, die Wirkungsdauer, die beiden ordinalen Schmerz-Parameter (Intensität und Nachlassung des Schmerzes), den Rängen des spätesten Wirkungseintritts und spätesten Wirkungsverlusts und der Wirksamkeits- und Verträglichkeits-Einschätzung wurden ELEMENTARSTATISTIKen ermittelt.

Für die patientenbeschreibenden Binär-Parameter und Nebenwirkungen bei bestimmungsgemäßer Anwendung wurden für den Vergleich der beiden Patienten-Gruppen VIERFELDER-TESTE nach FISHER (Statistische Methoden für die Wissenschaft, London: Oliver-Boyd; 1956) eingesetzt. Der Gruppen-Vergleich bezüglich der metrischen patienten- und versuchsbeschreibenden Parameter erfolgte mit dem U-TEST nach MANN-WHITNEY (Ann. Math. Stat. 1947; 18: 50-60). Für den zufallskritischen Vergleich der beiden Patienten-Gruppen über die 3 Untersuchungstage wurden für die Dosierung, Schmerz-Intensität, Nachlassen des Schmerzes, Rang des spätesten Wirkungseintritts und Wirkungsverlust, Mindestwirkzeit und Wirkungsdauer sowie die Wirksamkeitsund Verträglichkeits-Einschätzung des Prüfarztes SIMULTANE U-TESTE mit ALPHA-Adjustierung eingesetzt (Buck, EDV in Medizin und Biologie 1976; 7: 65-75).

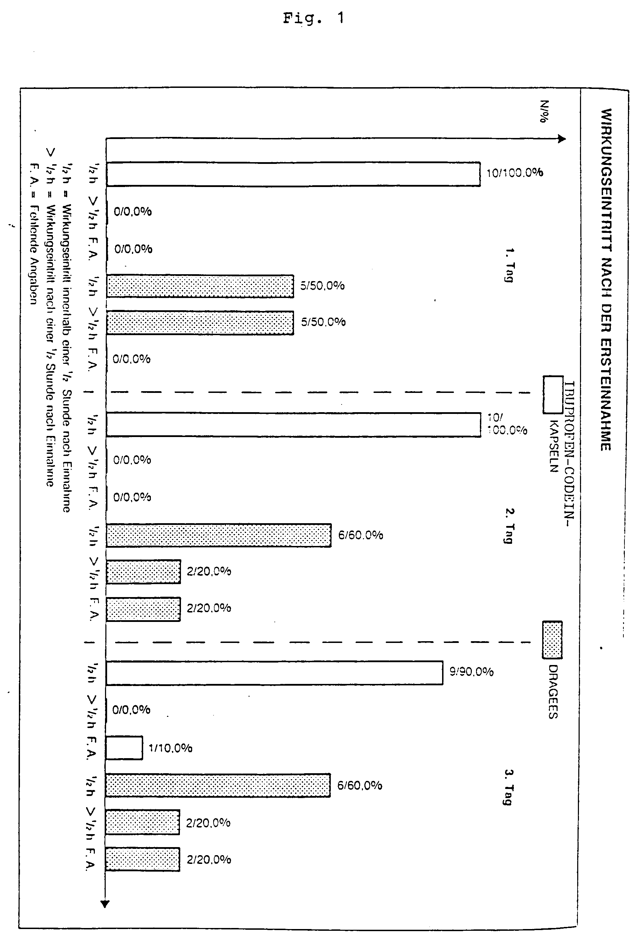

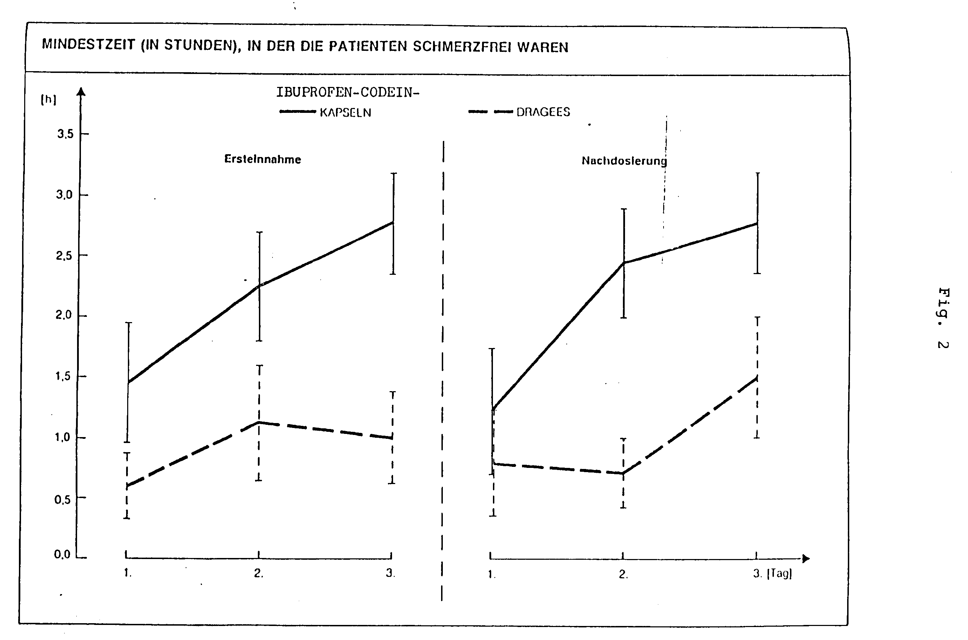

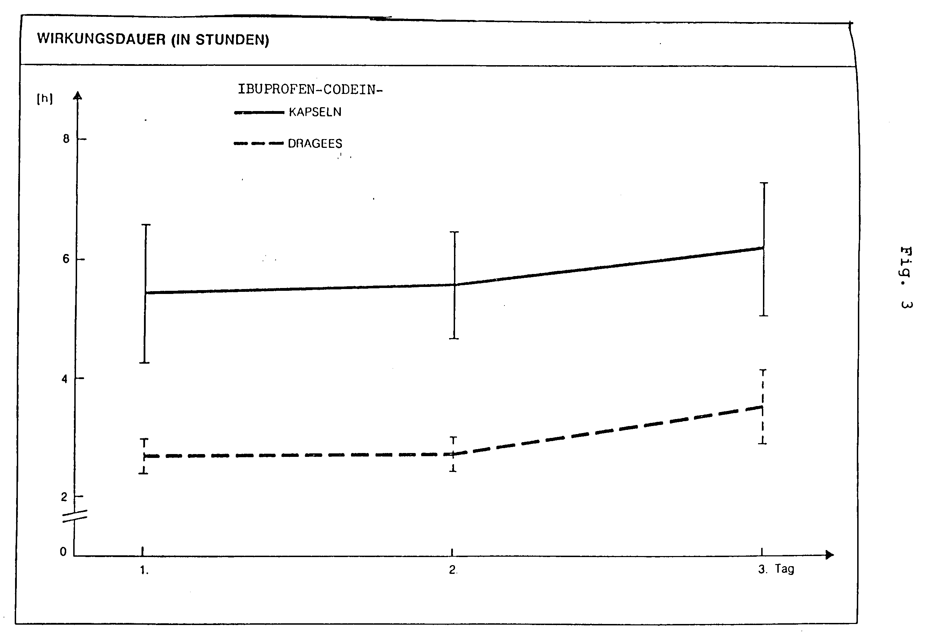

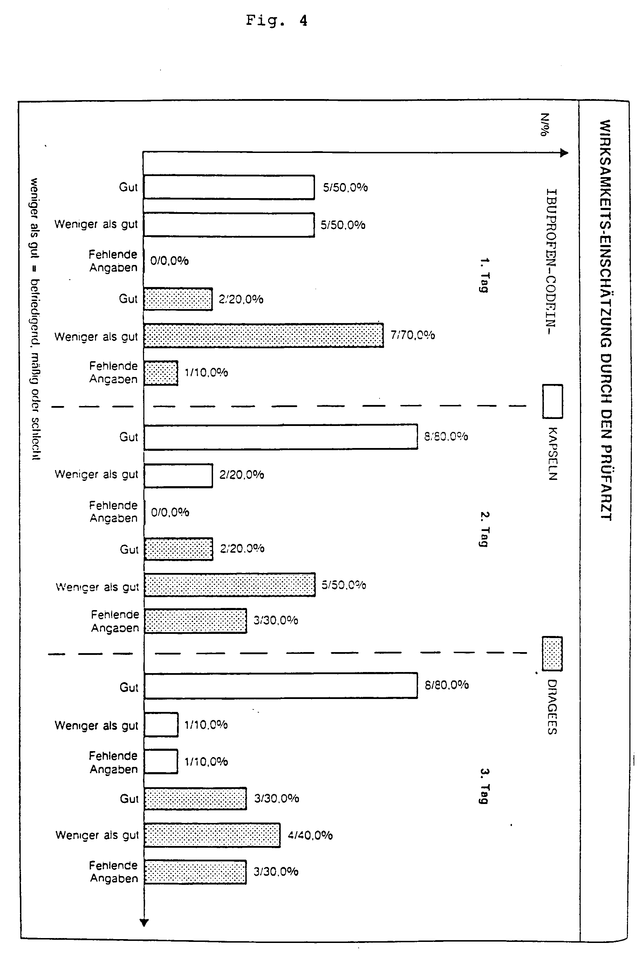

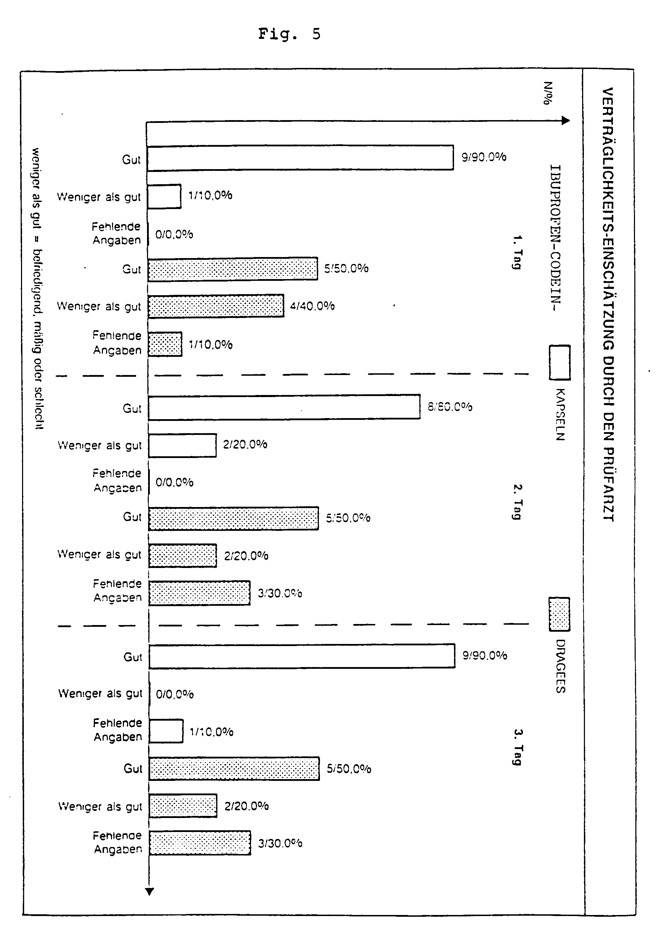

Die ermittelten Unterschiede in der Wirksamkeit zwischen den Patientengruppen sind statistisch signifikant. Die Ergebnisse sind in den Figuren 1 bis 5 zusammengestellt und graphisch dargestellt.

## Beispiel 3

Die gemäß Beispiel 1 hergestellten Weichgelatinekapseln wurden in einer offenen Wirksamkeitsstudie bei 26 Patienten mit schweren postoperativen Schmerzzuständen geprüft. Die Indikationen für die Operation waren:

Gonarthrose
Nekrose am Fuß nach Sprunggelenkfraktur
Metallentfernung am Sprunggelenk
Endoprothese
Knieendoprothese
Hammerzehe
Exostosenabmeisselung
Meniskotomie
Arthrodese OP-Sprunggelenk
Radiusfraktur
Bursitis Knie-Extirpation
Medialer Schlitten
Ellengelenksrevision

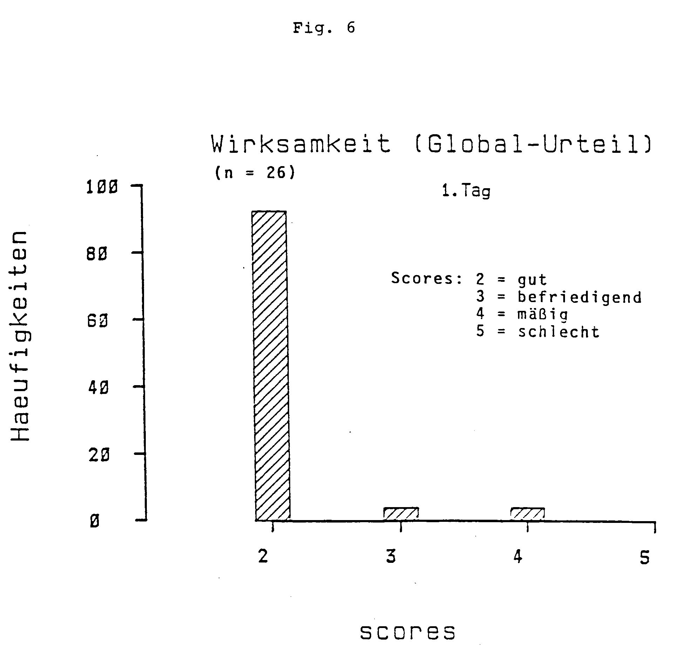

Die Patienten erhielten 3mal 1 Kapsel pro Tag. Die Wirkung hielt 3 bis 10 Stunden (durchschnittlich 5,35 Stunden) an. Die Ergebnisse sind in den Tabellen 1 und 2 sowie in Fig. 6 dargestellt:

Tabelle 1

Schmerzbeurteilung (Schm) 1. Tag   (n = 26)

Skore-Werte: 0 = nicht vorhanden
             1 = leicht
             2 = mäßig
             3 = stark
             4 = sehr stark

m. V. = missing values

| Skores | 0 | 1 | 2 | 3 | 4 | m.V. |
|---|---|---|---|---|---|---|
| Schm v. Th. | 0 | 0 | 1 | 24 | 0 | 1 |
| Schm n. 1/2 h | 4 | 15 | 4 | 2 | 0 | 1 |
| Schm n. 1 h | 7 | 14 | 2 | 2 | 0 | 1 |
| Schm n. 2 h | 8 | 12 | 3 | 2 | 0 | 1 |
| Schm n. 3 h | 8 | 10 | 5 | 2 | 0 | 1 |
| Schm n. 4 h | 7 | 11 | 5 | 1 | 0 | 2 |

Tabelle 2

Nachlassen der Schmerzen (NSchm) 1. Tag
(n = 26)

Skore-Werte: 1 = völlig
2 = deutlich
3 = geringgradig
4 = gar nicht

m. V. = missing values

| Skores | 1 | 2 | 3 | 4 | m.V. |
|---|---|---|---|---|---|
| NSchm n. 1/2 h | 4 | 16 | 4 | 2 | 0 |
| NSchm n. 1 h | 7 | 15 | 2 | 2 | 0 |
| NSchm n. 2 h | 8 | 14 | 2 | 2 | 0 |
| NSchm n. 3 h | 8 | 9 | 7 | 2 | 0 |
| NSchm n. 4 h | 7 | 12 | 5 | 1 | 1 |

**Beispiel 4**

Um festzustellen, in welcher Weise Ibuprofen und Codein bei erfindungsgemäßer Zubereitung wirken, wurden weitere Humanversuche durchgeführt.

Dem Versuchsbericht Teil A ist entnehmbar, daß Tenside die Verfügbarkeit eines jeden Wirkstoffs nicht gleichermaßen verändern. Der Codeinplasmaspiegel ist bei den erfindungsgemäßen Kapseln bereits nach etwa 2 Stunden auf etwa die Hälfte zurückgegangen, während bei den Dragees dies erst nach ca. 4 Stunden geschehen ist. Ähnlich verläuft der Plasmaspiegel des Ibuprofen in der erfindungsgemäßen Kapsel, während sich beim Dragee eine flachere Kurve des Plasmaspiegels von Ibuprofen ausbildet, so daß hier erst nach ca. 4 1/2 Stunden der Plasmaspiegel auf die Hälfte abgesunken ist. Bemerkenswert ist, daß der Plasmaspiegel beider Komponenten gemäß der Erfindung synchronisiert ist, während beim Dragee in der Anfangsphase wenig kongruente Kurvenverläufe zu beobachten sind. Wie aus den Plasmaspiegelkurven und den $C_{max}$-Werten zu ersehen ist, ergibt sich für Ibuprofen in der Anfangsphase bei den erfindungsgemäßen Kapseln eine bessere Verfügbarkeit als bei den Dragees. Die AUC-Werte dagegen sagen aus, daß die Bioverfügbarkeit des Ibuprofens über die beobachtete Zeit innerhalb der Fehlergrenze für beide Präparate gleich ist. Beim Codein zeigen sowohl die Plasmaspiegelkurven als auch die ermittelten AUC- und $C_{max}$-Werte keinen Unterschied hinsichtlich der Bioverfügbarkeit an. Der etwas höhere AUC-Wert des Codeins bei den Dragees deutet sogar eine bessere Verfügbarkeit an. Der Unterschied der AUC-Werte beider Formen ist jedoch statistisch nicht signifikant.

Im Fall der erfindungsgemäßen Kapseln wird zwar die Resorption des Ibuprofens im Vergleich zum Dragee in der Anfangsphase unmittelbar nach der Einnahme beschleunigt, die Verfügbarkeit des Codeins dagegen nicht beeinflußt. Der langsamere Abfall der Plasmaspiegelkurven der Dragees gegenüber den Kapseln würde daher sogar eine bessere Wirksamkeit und vor allem eine längere Dauer der Wirkung der Dragees gegenüber den Kapseln erwarten lassen. Die Befunde des erfindungsgemäßen Beispiels 2 ergaben jedoch überraschenderweise gerade das Gegenteil, nämlich eine bessere und länger anhaltende Verminderung des Schmerzes nach Applikation der erfindungsgemäßen Kapseln. Diese Befunde zeigen, daß im Fall der Kombination von Ibuprofen und Codein in Kapseln gemäß der Erfindung die überraschend gute Wirkung bei der Bekämpfung des Schmerzes nicht allein auf den Zusatz von Tensiden zurückgeführt werden kann. Hier sind offensichtlich noch unerwartete metabolische und synergistische Effekte zwischen wirksamen und nicht wirksamen Stoffen im Spiel.

Teil B des Versuchsberichtes zeigt einen weiteren Vorteil bei der Verwendung der erfindungsgemäßen Kapseln. Cooper et al. (Pharmacotherapy, 1982, 2, 162 - 167) haben beobachtet, daß die Kombination von 400 mg Ibuprofen + 60 mg Codein geringfügig, jedoch statistisch nicht signifikant, besser analgetisch wirksam sind als 400 mg Ibuprofen allein. Nach Sunshine et al. loc. cit. ist nur eine hohe Dosis von 400 mg Ibuprofen und 60 mg Codein der Einzelgabe von 400 mg Ibuprofen bei der Schmerzbekämpfung überlegen. Niedrige Dosierung von Codein (30 mg) und Ibuprofen zeigt keine Verbesserung gegen Ibuprofen allein. Wird jedoch die Wirkstoffkombination in Form der erfindungsgemäßen Kapseln gegeben, so bewirkt bereits der geringe Zusatz von 25 mg Codein eine statistisch signifikante, bessere analgetische Wirkung als mit Ibuprofen allein. Auch dieser Befund zeigt eindeutig, daß bei Verwendung der erfindungsgemäßen Kapseln überraschende Effekte auftreten.

## TEIL A

Vergleich der Bioverfügbarkeit der Wirkstoffe aus Ibuprofen-Codein-Kapseln (Erfindung Beispiel 1) und Ibuprofen-Codein-Dragées.

Die Studie war als offener Versuch angelegt. Die verwendeten Kapseln bzw. Dragées enthielten jeweils 400 mg Ibuprofen + 25 mg Codeinphosphat. An der Studie nahmen insgesamt 14 gesunde freiwillige männliche Versuchspersonen teil (6 bei den Dragées, 8 bei den Kapseln). Den Probanden wurde jeweils 1 mal eines der Präparate verabreicht, nach bestimmten Zeiten Blut entnommen und darin der Ibuprofen- bzw. Codein-Gehalt bestimmt. Die Mittelwerte dieser Plasmabestimmungen sind in der Abbildung in Abhängigkeit von der Zeit graphisch dargestellt. Aus diesen Mittelwertkurven wurden die für die Beurteilung der Bioverfügbarkeit entscheidenden Parameter berechnet: AUC = Fläche unter der Plasmaspiegelkurve als Maß für die über die beobachtete Zeit im Organismus vorhandene Substanzmenge und $C_{max}$ = maximale Plasmakonzentration der Wirkstoffe. Die Berechnungen erfolgten auf einem Commodore cbm 3032, die der AUC mittels der Trapezregel.

<u>K a p s e l n</u> (erfindungsgemäß):

I b u p r o f e n :
AUC $= 98,5$ h.µg/ml
$C_{max} = 35,9$ µg/ml
C o d e i n :
AUC $= 180,9$ h.ng/ml
$C_{max} = 70,8$ ng/ml

<u>D r a g é e s :</u>

I b u p r o f e n :
AUC $= 106,5$ h.µg/ml
$C_{max} = 27,9$ µg/ml
C o d e i n :
AUC $= 217,2$ h.ng/ml
$C_{max} = 68,7$ ng/ml

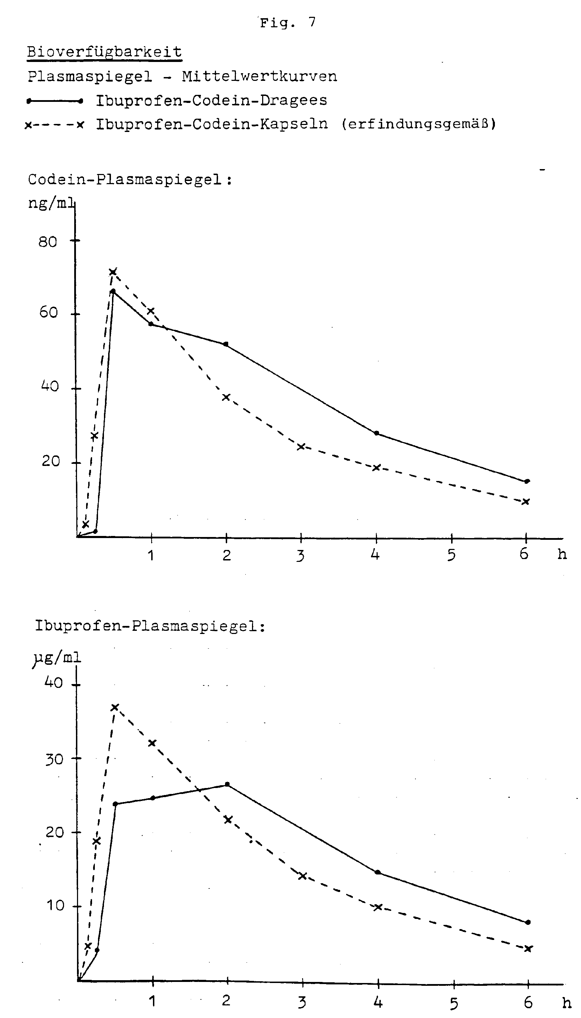

Die Ergebnisse sind in Fig. 7 zusammengesellt.

## TEIL B

Vergleich der Wirksamkeit der erfindungsgemäßen Ibuprofen-Codein-Kapseln gemäß Beispiel 1 mit Ibuprofen-Kapseln entspr. EP 178 436 (Beispiel 19).

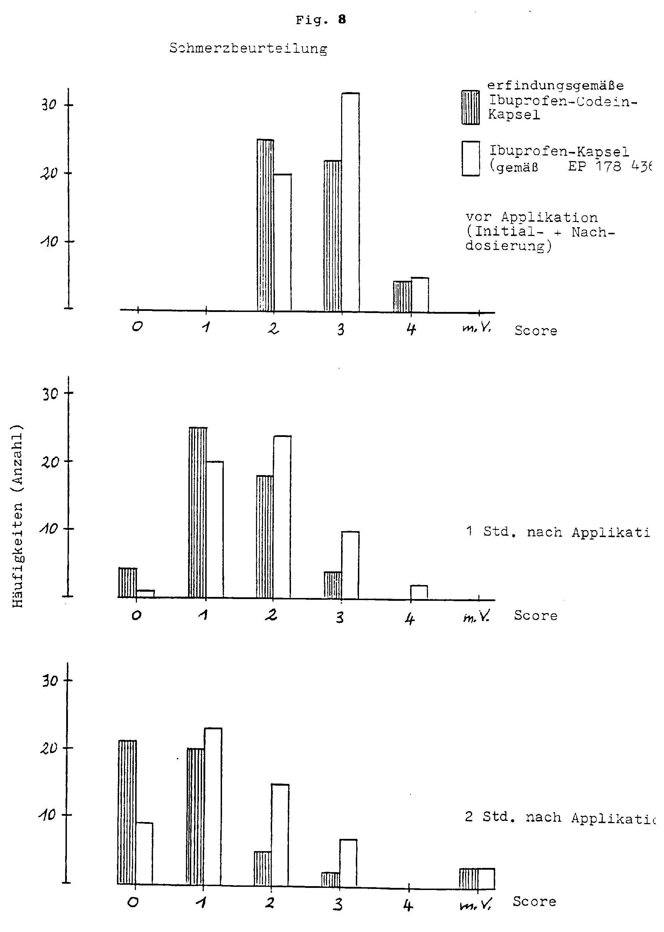

Die Studie war als offener cross-over Versuch mit randomisierter Zuordnung der Präparate angelegt. Die in der Prüfung verwendeten erfindungsgemäßen Kapseln enthielten 400 mg Ibuprofen + 25 mg Codeinphosphat bzw. 400 mg Ibuprofen gemäß EP 178 436). An der Studie nahmen 16 Patienten mit Periarthritis humero scapularis, Lumbago oder HWS- bzw. Arm-Schulter-Syndrom teil. Am Versuchstag wurde initial eine Kapsel verabreicht und dann nach Abklingen der Wirkung mit je 1 Kapsel nachdosiert. Auf diese Weise wurden 2 bis 4 mal 1 Kapsel im Abstand von 3 bis 6 Stunden innerhalb der Versuchsdauer von 12 Stunden eingenommen. Jeweils unmittelbar vor und 1 bzw. 2 Stunden nach der Einnahme wurde der Schmerzzustand abgefragt und nach folgendem Score beurteilt (Fig. 8):

0 = nicht vorhanden

1 = leicht

2 = mäßig

3 = stark

4 = sehr stark

m.V. = missing Value

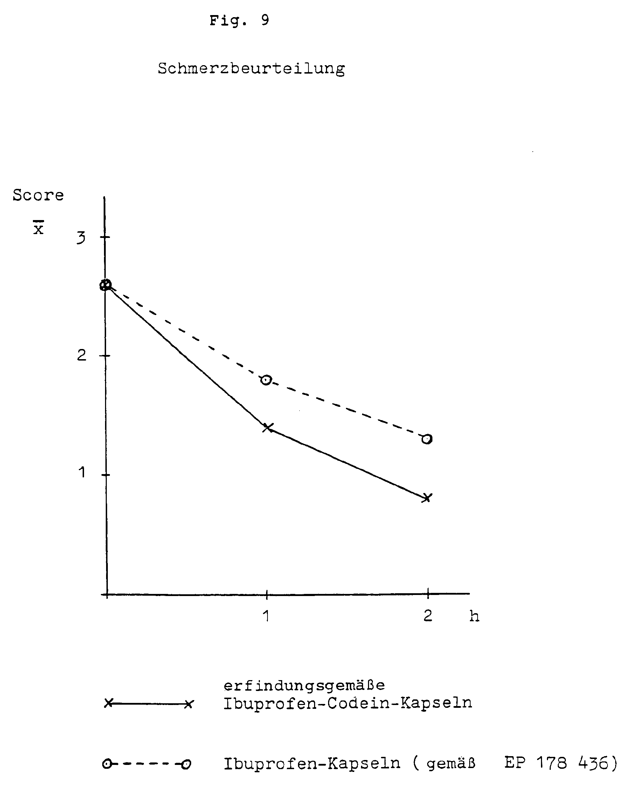

Aus den Einzel-scores wurden für jeden Patienten und jeden Zeitpunkt die Durchschnittswerte gebildet (Schmerzintensität; Tabelle 3). Schließlich wurden mittels der Trapezregel die Wirksamkeitsintegrale (= Flächen unter den Score-Zeit-Kurven) berechnet, die ein Maß für die Schmerzintensität über die beobachtete Zeit darstellen. Die Wirksamkeitsintegrale wurden auf einem Commodore cbm 3032 berechnet. Der Unterschied der mittleren Wirksamkeitsintegrale ($\bar{x}$) zwischen den Ibuprofen-Codein-Kapseln und den Ibuprofen-Kapseln ist signifikant (einseitiger U-Test nach Wilcoxon, Mann u. Whitney; Fig. 9). Aus der Anzahl der erforderlichen Nachdosierungen können außerdem Rückschlüsse auf eine längere Wirkungsdauer der Ibuprofen-Codein- gegenüber den Ibuprofen-Kapseln gezogen werden (Tabelle 3).

Die Befunde der vorliegenden Studie zeigen deutlich, daß die erfindungsgemäßen Ibuprofen-Codein-Kapseln hinsichtlich Schmerzbeurteilung, Beeinflussung der Schmerzintensität und Wirkungsdauer den vorbekannten Ibuprofen-Kapseln (gemäß EP 178 436) überlegen sind.

Tabelle 3 : Schmerzbeurteilung

| Patient (Initialen) | erfindungsgemäße Ibuprofen-Codein-Kapsel Schmerzintensität (Score-Durchschnittswert) | | | Wirkungs-integral | Ibuprofen-Kapsel (gemäß EP 178 436) Schmerzintensität (Score-Durchschnittswert) | | | Wirkungs-integral |
| --- | --- | --- | --- | --- | --- | --- | --- | --- |
| | nach 0 | 1 | 2 h | (h.score) | nach 0 | 1 | 2 h | (h.score) |
| 1 (HM) | 3 | 1,7 | 1 | 3,7 | 3 | 1 | 1 | 3,0 |
| 2 (TK) | 2 | 1,7 | 1 | 3,2 | 2 | 1,3 | 1,3 | 2,9 |
| 3 (CH) | 2,8 | 0,7 | 0 | 2,1 | 2,8 | 2,7 | 1,7 | 5,0 |
| 4 (PA) | 3 | 1,3 | 1,3 | 3,4 | 3 | 2 | 2 | 4,5 |
| 5 (NE) | 2 | 0,7 | 0 | 1,7 | 2 | 1,5 | 1,3 | 3,2 |
| 6 (BM) | 3 | 2 | 2,2 | 4,6 | 3 | 2 | 1,5 | 4,3 |
| 7 (PT) | 2,5 | 1 | 0 | 2,2 | 2,5 | 0,7 | 0,5 | 2,2 |
| 8 (HT) | 2,5 | 0,7 | 0 | 1,9 | 2,5 | 1,7 | 0,7 | 3,3 |
| 9 (AN) | 3,4 | 1,7 | 1,3 | 4,0 | 3,4 | 3,2 | 2,7 | 6,2 |
| 10 (PB) | 2,5 | 1 | 0,7 | 2,6 | 2,5 | 1,7 | 1 | 3,5 |
| 11 (AT) | 2,6 | 1,5 | 0,7 | 3,1 | 2,6 | 1,7 | 1 | 3,5 |
| 12 (HS) | 2 | 1 | 0 | 2,0 | 2 | 1,7 | 1 | 3,2 |
| 13 (KC) | 2,3 | 2 | 1 | 3,6 | 2,3 | 2 | 2 | 4,2 |
| 14 (DA) | 3,7 | 2,7 | 2 | 5,5 | 3,7 | 3 | 3 | 6,4 |
| 15 (JB) | 2,3 | 1,7 | 0,3 | 3,0 | 2,3 | 2 | 0 | 3,1 |
| 16 (AH) | 2,5 | 1,3 | 0,5 | 2,8 | 2,5 | 1 | 0 | 2,3 |
| x̄ | 2,6 | 1,4 | 0,8 | 3,1 | 2,6 | 1,8 | 1,3 | 3,8 |
| Nachdosie-rungen (Anzahl) | 35 | | | | 41 | | | |

EP 0 413 171 B1

**Patentansprüche**

1. Mittel zur Behandlung von schweren Schmerzzuständen, insbesondere von Tumorschmerzen und postoperativen Schmerzen bestehend aus Weichgelatinekapseln enthaltend 30 bis 50 Gewichtsteile Ibuprofen und 1,5 bis 4 Gewichtsteile Codein und/oder seine physiologisch verträglichen Salze, teilweise gelöst und teilweise suspendiert in 68,5 bis 46 Gewichtsteilen Polyoxyethylen-polyoxypropylen-Diol oder einem Gemisch aus 30 bis 76 Gewichtsteilen Polyoxyethylen-polyoxypropylen-Diol oder Polyethylenglykol oder Polypropylenglykol und 7 bis 40 Gewichtsteilen eines physiologisch verträglichen Tensids.

2. Mittel gemäß Anspruch 1, dadurch gekennzeichnet, daß sie zusätzlich bis zu 3 Gewichtsteile 1,2-Propylenglykol enthalten.

3. Mittel gemäß einem der Ansprüche 1 oder 2, dadurch gekennzeichnet, daß sie als Tensid Polyoxyethylen-glycerol-tri-hydroxystearat, Polyoxyethylen-($C_{12-18}$)-fettalkoholether, Polyoxyethylen-stearat, Polyoxyethylen-sorbitan-mono($C_{12-18}$)-fettsäureester, Polyoxyethylen-polyoxypropylen-Diol oder Gemische derselben enthalten.

4. Mittel gemäß einem der Ansprüche 1 bis 3, dadurch gekennzeichnet, daß sie 40 bis 45 Gewichtsteile Ibuprofen und 2,5 bis 3 Gewichtsteile Codeinphosphat·0,5 $H_2O$ enthalten, welche teilweise gelöst und teilweise suspendiert sind, in einem Gemisch aus 86 bis 90 Gewichtsteilen Polyoxyethylen-polypropylen-Diol, 8 bis 12 Gewichtsteilen Polyoxyethylen-(40)-glycerol-tri-hydroxystearat und 1,5 bis 2,5 Gewichtsteilen Propylenglykol.

5. Verfahren zur Herstellung von Mitteln zur Behandlung von schweren Schmerzzuständen, insbesondere von Tumorschmerzen und postoperativen Schmerzen, dadurch gekennzeichnet, daß 15 bis 30 Gewichtsteile Ibuprofen bei Temperaturen zwischen 45 und 65°C in 70 bis 85 Gewichtsteilen Polyoxyethylen-Polyoxypropylen-Diol oder in einem Gemisch aus 30 bis 76 Gewichtsteilen Polyoxyethylen-polyoxypropylen-Diol oder Polyethylenglykol oder Polypropylenglykol und 7 bis 40 Gewichtsteilen eines physiologisch verträglichen Tensids gelöst, auf Raumtemperatur abgekühlt, in der Lösung nach dem Abkühlen zusätzlich bis zu 40 Gewichtsteile Ibuprofen und 1,5 bis 4 Gewichtsteile Codein und/oder seine physiologisch verträglichen Salze suspendiert werden und das Gemisch in an sich bekannter Weise in Weichgelatinekapseln eingearbeitet wird.

6. Verfahren gemäß Anspruch 5, dadurch gekennzeichnet, daß den Lösungsmitteln bis zu 3 Gewichtsteilen 1,2-Propylenglykol zugesetzt werden.

7. Verfahren gemäß einem der Ansprüche 5 oder 6, dadurch gekennzeichnet, daß als Tensid Polyoxyethylen-glycerol-tri-hydroxystearat, Polyoxyethylen-($C_{12-18}$)-fettalkoholether, Polyoxyethylen-stearat, Polyoxyethylensorbitan-mono-($C_{12-18}$)-fettsäureester, Polyoxyethylen-polyoxypropylen-Diol oder Gemische derselben verwendet werden.

8. Verfahren gemäß einem der Ansprüche 5 bis 7, dadurch gekennzeichnet, daß zunächst 20 bis 25 Gewichtsteile Ibuprofen in einem warmen Gemisch aus 86 bis 90 Gewichtsteilen Polyoxyethylen-polyoxypropylen-Diol, 8 bis 12 Gewichtsteilen Polyoxyethylen-(40)-glycerol-tri-hydroxystearat und 1,5 bis 2,5 Gewichtsteilen 1,2-Propylenglykol gelöst, dann auf Raumtemperatur abgekühlt und in dem Gemisch weitere 20 bis 25 Gewichtsteile Ibuprofen und 2,5 bis 3 Gewichtsteile Codeinphosphat·0,5 $H_2O$ suspendiert werden und in Weichgelatinekapseln abgefüllt werden.

**Claims**

1. Agents for the treatment of conditions of severe pain, and especially of tumor pain and postoperative pain, consisting of soft gelatin capsules containing from 30 to 50 parts by weight of ibuprofen and from 1.5 to 4 parts by weight of codein and/or physiologically compatible salts thereof, partially dissolved and partially suspended in 68.5 to 46 parts by weight of polyoxyethylene-polyoxypropylene-diol or in a mixture comprising 30 to 76 parts by weight of polyoxyethylene-polyoxypropylene-diol or polyethyleneglycol or polypropyleneglycol and from 7 to 40 parts by weight of a physiologically compatible surfactant.

2. The agents according to claim 1, characterized in that they contain up to 3 parts by weight of 1,2-propy-

EP 0 413 171 B1

leneglycol.

3. The agents according to any of claims 1 or 2, characterized in that they contain polyoxyethyleneglycerol-tri-hydroxystearate, polyoxyethylene-($C_{12-18}$)-fatty alcohol ether, polyoxyethylene-stearate, polyoxyethylene-sorbitan-mono($C_{12-18}$)-fatty acid ester, polyoxyethylene-polyoxypropylene-diol or mixtures thereof as a surfactant.

4. The agents according to any of claims 1 to 3, characterized in that they contain from 40 to 45 parts by weight of ibuprofen and from 2.5 to 3 parts by weight of codeine phosphate · 0.5 $H_2O$, which in part have been dissolved and in part have been suspended in a mixture comprising from 86 to 90 parts by weight of polyoxyethylene-polyoxypropylene-diol, 8 to 12 parts by weight of polyoxyethylene(40)-glycerol-tri-hydroxystearate and from 1.5 to 2.5 parts by weight of propyleneglycol.

5. A process for preparing agents for the treatment of conditions of severe pain, and especially of tumor pain and postoperative pain, characterized in that from 15 to 30 parts by weight of ibuprofen are dissolved in 70 to 85 parts by weight of polyoxyethylene-polyoxypropylene-diol or in a mixture comprising 30 to 76 parts by weight of polyoxyethylene-polyoxypropylene-diol or polyethyleneglycol or polypropyleneglycol and 7 to 40 parts by weight of a physiologically compatible surfactant at temperatures of between 45 °C and 65 °C, are allowed to cool to room temperature, in addition up to 40 parts by weight of ibuprofen and 1.5 to 4 parts by weight of codeine and/or its physiologicaly compatible salts are suspended in the solution after cooling, and the mixture is incorporated in soft gelatin capsules in a <u>per se</u> known manner.

6. The process according to claim 5, characterized in that up to 3 parts by weight of 1,2-propyleneglycol are added to the solvents.

7. The process according to any of claims 5 or 6, characterized in that polyoxyethylene-glycerol-trihydroxystearate, polyoxyethylene-($C_{12-18}$)-fatty alcohol ether, polyoxyethylene-stearate, polyoxyethylene-sorbitan-mono($C_{12-18}$)-fatty acid ester, polyoxyethylene-polyoxypropylene-diol or mixtures thereof are used as the surfactant.

8. The process according to any of claims 5 to 7, characterized in that first 20 to 25 parts by weight of ibuprofen are dissolved in a warm mixture of from 86 to 90 parts by weight of polyoxyethylene-polyoxypropylene-diol, 8 to 12 parts by weight of polyoxyethylene(40)-glycerol-tri-hydroxystearate and from 1.5 to 2.5 parts by weight of 1,2-propyleneglycol, then the obtained mixture is allowed to cool down to room temperature, and from 20 to 25 parts by weight of ibuprofen and from 2.5 to 3 parts by weight of codeine phosphate · 0.5 $H_2O$ are further suspended in said mixture, and then the resultant mixture is filled into soft gelatin capsules.

**Revendications**

1. Produits pour le traitement d'états douloureux graves, en particulier de douleurs tumorales et de douleurs postopératoires, formés de capsules de gélatine molle contenant de 30 à 50 parties en poids d'ibuprofène et 1,5 à 4 parties en poids de codéine et/ou de ses sels physiologiquement acceptables, en partie dissous et en partie en suspension dans 68,5 à 46 parties en poids de polyoxyéthylène-polyoxypropylène-diol ou dans un mélange de 30 à 76 parties en poids de polyoxyéthylène-polyoxypropylène-diol ou de polyéthylèneglycol ou de polypropylèneglycol et 7 à 40 parties en poids d'un agent tensio-actif physiologiquement acceptable.

2. Produits selon la revendication 1, caractérisés en ce qu'ils contiennent, en outre, jusqu'à 3 parties en poids de 1,2-propylèneglycol.

3. Produits selon la revendication 1 ou 2, caractérisés en ce qu'ils contiennent, comme agent tensio-actif, le trihydroxystéarate de polyoxyéthylèneglycérol, un éther de polyoxyéthylène et d'alcool gras en $C_{12-18}$, le stéarate de polyoxyéthylène, un ester de polyoxyéthylènesorbitanne et d'un mono-acide gras en $C_{12-18}$, un polyoxyéthylène-polyoxypropylène-diol, ou des mélanges de ces substances.

4. Produits selon une des revendications 1 à 3, caractérisés en ce qu'ils contiennent de 40 à 45 parties en poids d'ibuprofène et 2,5 à 3 parties en poids de phosphate de codéine.0,5 $H_2O$, qui sont en partie dissous

10

et en partie en suspension, dans un mélange de 86 à 90 parties en poids de polyoxyéthylène-polypropylène-diol, 8 à 12 parties en poids de trihydroxystéarate de polyoxyéthylène-(40)-glycérol et 1,5 à 2,5 parties en poids de propylèneglycol.

5. Procédé de préparation de produits pour le traitement d'états douloureux graves, en particulier de douleurs tumorales et de douleurs postopératoires, caractérisé en ce qu'on dissout 15 à 30 parties en poids d'ibuprofène, à une température comprise entre 45 et 65°C, dans 70 à 85 parties en poids de polyoxyéthylène-polyoxypropylène-diol ou dans un mélange de 30 à 76 parties en poids de polyoxyéthylène-polyoxypropylène-diol ou de polyéthylène-glycol ou de polypropylène-glycol et de 7 à 40 parties en poids d'un agent tensio-actif physiologiquement acceptable, on refroidit le mélange à la température ambiante et après le refroidissement, on met, en outre, jusqu'à 40 parties en poids d'ibuprofène et 1,5 à 4 parties de codéine et/ou de ses sels physiologiquement acceptables en suspension dans la solution et on introduit le mélange, de manière connue en soi, dans des capsules de gélatine molle.

6. Procédé selon la revendication 5, caractérisé en ce qu'on ajoute jusqu'à 3 parties en poids de 1,2-propylèneglycol aux solvants.

7. Procédé selon la revendication 5 ou 6, caractérisé en ce qu'on utilise, comme agent tensio-actif, le trihydroxystéarate de polyoxyéthylène-glycérol, un éther de polyoxyéthylène et d'alcool gras en $C_{12-18}$, le stéarate de polyoxyéthylène, un ester de polyoxyéthylènesorbitanne et d'un mono-acide gras en $C_{12-18}$, un polyoxyéthylène-polyoxypropylène-diol, ou des mélanges de ces substances.

8. Procédé selon une des revendications 5 à 7, caractérisé en ce qu'on dissout d'abord 20 à 25 parties en poids d'ibuprofène dans un mélange chaud de 86 à 90 parties en poids de polyoxyéthylène-polyoxypropylène-diol, 8 à 12 parties en poids de trihydroxystéarate de polyoxyéthylène-(40)-glycérol et 1,5 à 2,5 parties en poids de 1,2-propylèneglycol, ensuite on refroidit le mélange à la température ambiante et on met, en outre, 20 à 25 parties en poids d'ibuprofène et 2,5 à 3 parties en poids de phosphate de codéine.0, 5 $H_2O$ en suspension dans le mélange et on introduit ce mélange dans des capsules de gélatine molle.

Fig. 1

WIRKUNGSEINTRITT NACH DER ERSTEINNAHME

N/%

IBUPROFEN-CODEIN-KAPSELN

DRAGEES

1. Tag
- ½ h — 10/100,0%
- > ½ h — 0/0,0%
- F.A. — 0/0,0%
- ½ h — 5/50,0%
- > ½ h — 5/50,0%
- F.A. — 0/0,0%

2. Tag
- ½ h — 10/100,0%
- > ½ h — 0/0,0%
- F.A. — 0/0,0%
- ½ h — 6/60,0%
- > ½ h — 2/20,0%
- F.A. — 2/20,0%

3. Tag
- ½ h — 9/90,0%
- > ½ h — 0/0,0%
- F.A. — 1/10,0%
- ½ h — 6/60,0%
- > ½ h — 2/20,0%
- F.A. — 2/20,0%

½ h = Wirkungseintritt innerhalb einer ½ Stunde nach Einnahme
> ½ h = Wirkungseintritt nach einer ½ Stunde nach Einnahme
F. A. = Fehlende Angaben

Fig. 2

MINDESTZEIT (IN STUNDEN), IN DER DIE PATIENTEN SCHMERZFREI WAREN

IBUPROFEN–CODEIN–
KAPSELN
DRAGEES

Erstelnnahme
Nachdoslerung

Fig. 3

EP 0 413 171 B1

Fig. 4

WIRKSAMKEITS-EINSCHÄTZUNG DURCH DEN PRÜFARZT

Fig. 5

Fig. 6

Wirksamkeit (Global-Urteil)
(n = 26)
1.Tag

Scores: 2 = gut
3 = befriedigend
4 = mäßig
5 = schlecht

scores

Haeufigkeiten

17

Fig. 7

Bioverfügbarkeit

Plasmaspiegel - Mittelwertkurven

●———● Ibuprofen-Codein-Dragees

×----× Ibuprofen-Codein-Kapseln (erfindungsgemäß)

Codein-Plasmaspiegel:

Ibuprofen-Plasmaspiegel:

Fig. 8

Schmerzbeurteilung

erfindungsgemäße
Ibuprofen-Codein-
Kapsel

Ibuprofen-Kapsel
(gemäß    EP 178 43€

vor Applikation
(Initial- + Nach-
dosierung)

1 Std. nach Applikati

2 Std. nach Applikati‹

Häufigkeiten (Anzahl)

Fig. 9

Schmerzbeurteilung

erfindungsgemäße
×——————× Ibuprofen-Codein-Kapseln

o-----o  Ibuprofen-Kapseln ( gemäß  EP 178 436)